# EUROPÄISCHE PATENTSCHRIFT

(11) **EP 0 481 310 B1**
(45) Veröffentlichungstag und Bekanntmachung des Hinweises auf die Patenterteilung: **07.12.1994**
(21) Anmeldenummer: 91117010.8
(22) Anmeldetag: 05.10.1991
(51) Int. Cl.: A61B 17/39

(54) **Schneidelektrode für medizinische Resektoskope**
Cutting electrode for medical resectoscope
Electrode de coupe pour résectoscope médical

(30) Priorität: 15.10.1990 DE 4032601
(43) Veröffentlichungstag der Anmeldung: 22.04.1992
(73) Patentinhaber: OLYMPUS WINTER & IBE GmbH, 22045 Hamburg (DE)
(72) Erfinder: Korth, Knut, Dr., W-7802 Merzhausen (DE); Nösel, Bernd, W-2073 Lütjensee (DE)
(74) Vertreter: Schaefer, Konrad, Dipl.-Phys.

(56) Entgegenhaltungen:
- DE-A- 2 521 719
- FR-A- 2 645 008

## Beschreibung

Die Erfindung betrifft eine Schneidelektrode der im Oberbegriff des Anspruches 1 genannten Art(vgl. z.B. DE-A-2521 719, Fig.7).

Derartige Schneidelektroden werden in der Medizin zum Entfernen von Gewebe verwendet, typischerweise in der Urologie und dort insbesondere zur Prostataresektion. Dabei ist die Schneidelektrode an einen Hochfrequenzgenerator angeschlossen, der mit einem Schalter vom Operateur ein- und ausgeschaltet werden kann. Bei angeschalteter Hochfrequenz schneidet die Schneidschlinge sehr leicht und fast ohne Widerstand durch das Körpergewebe. Das leichte, fast kraftfreie Schneiden mit einer derartigen Elektrode hat aber auch Nachteile. Der Operateur muß sich sehr auf die Operation konzentrieren und immer genau wissen, wie tief er schneiden kann. Eine unbedachte Bewegung oder Fehleinschätzung der möglichen Schneidtiefe führt zu ungewollten Verletzungen des Patienten.

In Ruhestellung steht bei üblicher Ausführung des Elektrodenantriebes eine derartige Schneidelektrode im Inneren des rohrförmigen Schaftes des Resektoskopes. Sie kann aus dem distalen Ende des Schaftes heraus nach vorn ausgefahren und dort in Achsrichtung hin- und herbewegt werten. Bei Einschalten des HF-Stromes kann dann unter Vor- oder Zurückbewegung der Elektrode mit deren Schneidschlinge geschnitten werden. Dabei ist in unmittelbarer Nähe des Resektoskopschaftes die mögliche Schneidtiefe durch die Auflage des Schaftes von selbst begrenzt. Je weiter sich die Schlinge aber vom Schaft entfernt, desto tiefer kann geschnitten werden, desto höher ist also das Risiko, zu tief zu schneiden. Außerdem besteht die Gefahr, nach vorn zu weit zu schneiden.

Die Aufgabe der vorliegenden Erfindung besteht darin, eine Schneidelektrode der eingangs genannten Art zu schaffen, mit der risikoärmer gearbeitet werden kann.

Diese Aufgabe wird erfindungsgemäß mit den Merkmalen des Kennzeichnungsteiles des Anspruches 1 gelöst.

Die erfindungsgemäße Schneidelektrode weist einen isolierten, also nicht schneidenden Distanzhalter auf, der vor der Schlinge angeordnet ist, also nach vorn Distanz hält und ein ungewolltes Eindringen nach vorn verhindert. Der Distanzhalter ragt ferner ein Stück nach unten und liegt dort beim Schneiden auf der Gewebeoberfläche auf, so daß die Schneidschlinge nur um das Stück, was sie tiefer ragt als der Distanzhalter, einschneiden kann. Die Hauptrisiken des ungewollten Durchschneidens nach vorn und des zu tiefen Schneidens nach unten werden also ausgeschlossen bzw. begrenzt. Mit einer derartigen Elektrode kann auch bei unkonzentriertem Arbeiten oder bei schlechter Sicht schnell gearbeitet werden, wobei Verletzungsgefahren für den Patienten weitgehend ausgeschlossen sind. Die erfindungsgemäße Schneidelektrode schützt also gegen Fehlschnitte aufgrund falscher Bewegungen des Operateurs. Gleichermaßen schützt die erfindungsgemäße Schneidelektrode auch gegen Fehlschnitte aufgrund unwillkürlicher Bewegungen des Patienten. Ein bekanntes Beispiel aus der Urologie ist der nervus obturatorius, der bei Arbeiten in der Blase gereizt werden kann und dann zu einer Reflexbewegung des Patienten führt, bei der es zu ungewollen Berührungen von Gewebe durch die Schneidschlinge kommt.

Vorteilhaft sind die Merkmale des Anspruches 2 vorgesehen. Eine solche Konstruktion ist kostengünstig herstellbar, wobei der Bügel auf einfache Weise weitgehend beliebige geometrisch erforderliche Formen annehmen kann.

Weiterhin vorteilhaft sind die Merkmale des Anspruches 3 vorgesehen. Durch die kufenartige Ausbildung der auf dem Gewebe aufsitzenden Teile des Distanzhalters werden gute Gleiteigenschaften erreicht, die das Schneiden nicht behindern.

Weiterhin vorteilhaft sind die Merkmale des Anspruches 4 vorgesehen. Auch auf diese Weise lassen sich mit rollender Auflage gute Gleiteigenschaften erreichen.

Weiterhin vorteilhaft sind die Merkmale des Anspruches 5 vorgesehen. Auf diese Weise ergibt sich eine stabile Zweipunktauflage der Elektrode, die der Schneidpräzision förderlich ist. Außerdem liegen auf diese Weise die Auflagepunkte seitlich neben der von der Elektrode geschnittenen Rinne, so daß diese die sichere Auflage nicht beeinflussen kann.

Weiterhin vorteilhaft sind die Merkmale des Anspruches 6 vorgesehen. Auf diese Weise wird freie Sicht des Operateurs zwischen Schlinge und Drahtbügel hindurch nach vorn gewährleistet.

In der Zeichnung ist die Erfindung beispielsweise und schematisch dargestellt. Es zeigen:
- Fig. 1: einen Schnitt durch die untere Hälfte einer Prostata mit erfindungsgemäßem Resektoskop im Schneideinsatz,
- Fig. 2: die in Fig. 1 dargestellte Elektrode in perspektivischer Ansicht,
- Fig. 3: einen vergrößerten Schnitt nach Linie 3 - 3 in Fig. 1,
- Fig. 4: die Seitenansicht einer zweiten Ausführungsform der Elektrode,
- Fig. 5: eine Achsansicht gemäß Fig. 3 der Elektrode der Fig. 4,
- Fig. 6: eine perspektivische Darstellung der Elektrode der Fig. 4 und 5,
- Fig. 7: eine Seitenansicht einer dritten Ausführungsform der Elektrode,
- Fig. 8: eine perspektivische Darstellung der Elektrode der Fig. 7 und
- Fig. 9: eine axiale Frontansicht der Ausführungsform der Figuren 7 und 8.

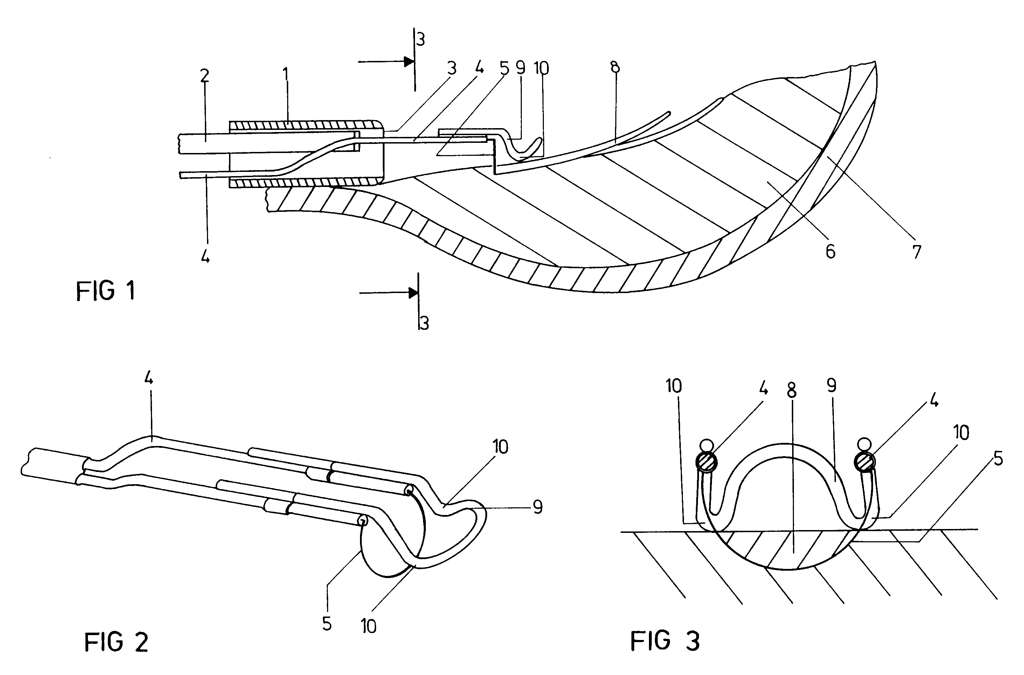

In Fig. 1 ist im Achsschnitt das distale Ende des Schaftes 1 eines Resektoskopes dargestellt. In diesem ist eine Optik 2 angeordnet, die nach vorn durch die vom Rand 3 begrenzte öffnung des Schaftes 1 blickt. Ferner ist im Schaft 1 eine Schneidelektrode angeordnet, bestehend aus einem Schlingenträger 4 mit einer Schneidschlinge 5.

Es ist eine Schneidelektrode handelsüblichen Typs dargestellt, die in ihrem längeren Teil aus einem Stab besteht, der sich zum distalen Ende hin gabelförmig verzweigt und zwischen den Gabelenden die im Halbkreis nach unten gebogene Schneidschlinge 5 aufweist. Die Schneidschlinge 5 besteht aus einem nicht isolierten Draht. Der Rest des Schlingenträgers 4 ist nach außen isoliert. Wenn Hochfrequenzstrom angelegt wird, tritt eine Schneidwirkung somit nur an der Schneidschlinge 5 auf.

Der Schlingenträger 4 ist in Achsrichtung des Schaftes 1 des Resektoskopes hin- und herbewegbar. Geschnitten wird üblicherweise bei festliegendem Schaft 1 durch Hin- und Herbewegung der Elektrode, wobei HF-Strom eingeschaltet ist. Üblicherweise wird rückziehend gearbeitet, wie dies in Fig. 1 dargestellt ist. Dort ist das Resektoskop in einer Prostata dargestellt mit zu entfernendem Prostatagewebe 6 in einer Kapsel 7, die nicht beschädigt werden darf.

Die insoweit beschriebene bekannte Elektrodenkonstruktion wird so eingesetzt, wie in Fig. 1 dargestellt. Bei rückziehender Bewegung wird ein Gewebestreifen 8 abgeschnitten, wie dies auch im Schnitt der Fig. 3 zu erkennen ist.

Dabei ist es erforderlich, durch Kippbewegung des Schaftes 1 die Schneidtiefe genau einzustellen. Da die Schneidschlinge 5 sehr leicht, fast kraftfrei durch Gewebe schneidet, kann ungewollt zu tief geschnitten werden. Insbesondere dann, wenn bereits viel vom Prostatagewebe 6 entfernt ist und in geringem Abstand zur Kapsel 7 geschnitten wird, kann ungewollt in diese eingeschnitten werden.

Besonders groß ist die Gefahr, geradeaus nach vorn in Verlängerung des Schaftes 1 zu weit vorzuschneiden und auch hier den Patienten zu verletzen, beispielsweise die Kapsel 7 zu perforieren.

Die erfindungsgemäße Konstruktion vermeidet dies durch Vorsehen eines Distanzhalters, der in der dargestellten Ausführungsform als Drahtbügel 9 ausgebildet ist. Der Drahtbügel besteht beispielsweise aus nach außen isoliertem Metalldraht und ist auf den beiden Gabelarmen des Schlingenträgers 4 befestigt, beispielsweise angeklebt oder angelötet. Er ragt, wie der Vergleich der Fig. 1 bis 3 zeigt, nach vorn über die Schneidschlinge 5 und verläuft dann abwärts gebogen bis zu einer Tiefe, die etwas geringer ist als die Tiefe der Schneidschlinge 5. Dort bei 10 ist der Drahtbügel kufenartig aufwärts gebogen. Wie Fig. 1 zeigt, liegt der Drahtbügel 9 also mit Gleitkufen auf dem Gewebe auf, wodurch leichtes reibungsfreies Gleiten auf der Gewebeoberfläche sichergestellt ist.

Die Fig. 1 und 3 zeigen, wie der Drahtbügel 9 mit seinen kufenartig geformten Auflagepunkten bei 10 auf der Gewebeoberfläche aufliegt und dadurch die Einschneidtiefe der Schneidschlinge 5 automatisch begrenzt, die, wie die Figuren zeigen, nicht tiefer eindringen kann als in den Figuren dargestellt. Auch bei unkonzentriertem Arbeiten kann die Schneidschlinge 5 nicht tiefer eindringen. Die Schneidtiefenbegrenzung ergibt sich sowohl bei rückwärtsschneidender als auch bei vorwärtsschneidender Resektionstechnik.

Der Drahtbügel 9 ergibt natürlich auch nach vorn eine sichernde Distanzwirkung. Wenn nach vorn irgendein Körperteil berührt wird, so erfolgt die Berührung auf jeden Fall mit dem nicht schneidenden Drahtbügel 9, so daß ungewollte Verletzungen vermieden werden.

Wie die Fig. 1 bis 3, insbesondere die Fig. 3, zeigen, ist der Drahtbügel zwischen den tiefsten Punkten bei 10 aufwärts gebogen. Der Operateur, der durch die Optik 2 in Achsrichtung des Schaftes blickt, sieht die Schneidschlinge 5 und den Drahtbügel 9 etwa so, wie in Fig. 3 dargestellt.

Durch die hochgebogene Ausführung des distalen Endes des Drahtbügels 9 ergibt sich ein freies Blickfeld für den Operateur zwischen Schneidschlinge 5 und Drahtbügel 9 hindurch, und zwar insbesondere auch dann, wenn diese Teile bei vollständig zurückgezogener Schneidelektrode unmittelbar vor der Optik 2 stehen.

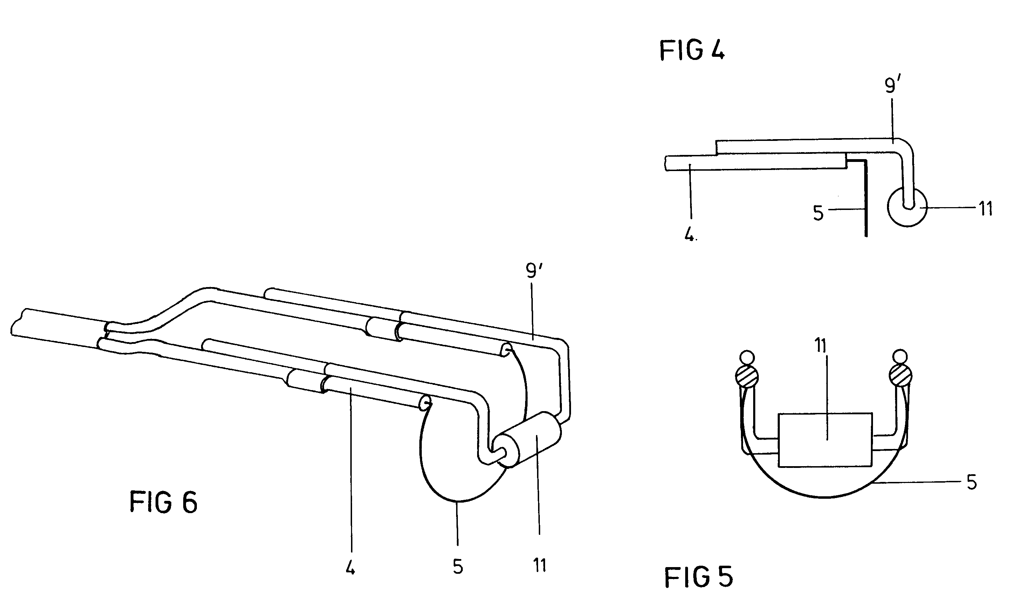

Die Fig. 4 bis 6 zeigen eine zweite Ausführungsform der Schneidelektrode, bei der der Schlingenträger 4 und die Schneidschlinge 5 identisch ausgebildet sind wie bei der Ausführungsform der Fig. 1 bis 3. Der Distanzhalter besteht wiederum aus einem Drahtbügel 9', der aber an seinem tiefsten Punkt eine Achse für eine Rolle 11 bildet. Diese stützt sich rollend auf der Oberfläche des zu schneidenden Gewebes, beispielsweise des Prostatagewebes 6, gemäß Fig. 1 ab und erlaubt ebenfalls bei sicherer Tiefenbegrenzung und Begrenzung nach vorn ein leichtes reibungsfreies Schneiden.

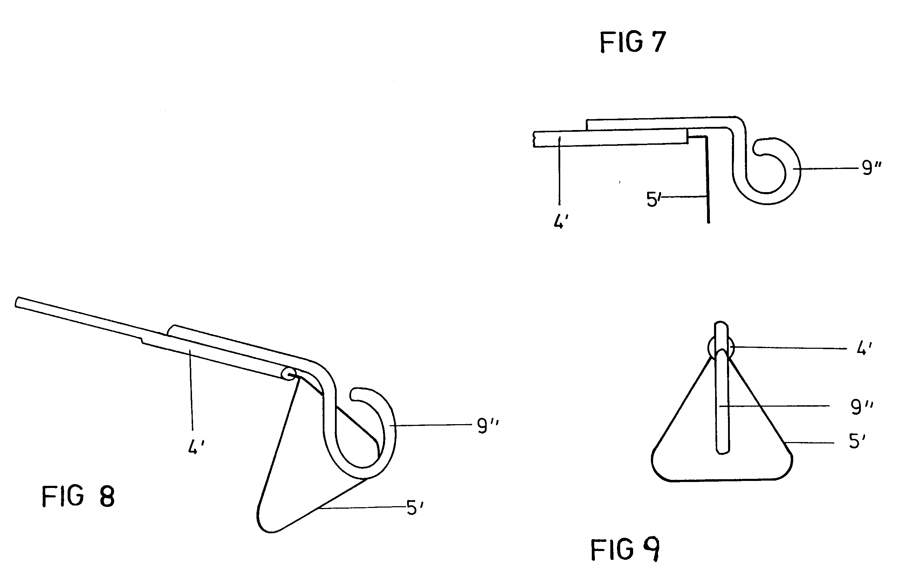

Die Fig. 7 bis 9 zeigen eine dritte Ausführungsform, bei der ein anderer Schlingenträger 4' vorliegt, der in seinem distalen Bereich nicht gegabelt ist und an dem eine geschlossene Schneidschlinge 5' sitzt, die von etwas anderer Formgebung ist. Es ist wiederum ein Drahtbügel 9'' vorgesehen, der jedoch nur mittig nach vorn und nach unten reicht und an seinem tiefsten Punkt wiederum kufenartig abgerundet ausgebildet ist.

An einem im vorderen distalen Endstück einfach ausgebildeten Schlingenträger 4' gemäß den Fig. 7 bis 9 kann jedoch auch ein Drahtbügel vorgesehen sein, der in seiner Formgebung dem der Fig. 1 bis 3 entspricht oder der eine Rolle aufweist gemäß den Fig. 4 bis 6. Die in den Figuren dargestellten Drahtbügelkonstruktionen 9, 9', 9'' kann aus isoliertem Metalldraht bestehen, aber auch aus geeignet festem, insbesondere warmfestem Kunststoff bestehen. Der Distanzhalter kann auch als geeignet geformter, räumlich geschlossener Körper ausgebildet sein.

Auch von den in den Figuren dargestellten Schneidschlingenkonstruktionen abweichende Schneidschlingenformen können in entsprechender Weise mit Distanzhaltern versorgt werden, bei denen es jeweils darauf ankommt, daß sie sich distal vor die Schneidschlinge und bis über einen gewissen Anteil von deren Tiefe erstrecken. Die tiefsten Stellen der Distanzhalter, mit denen sie auf dem Gewebe aufliegen, sind vorzugsweise gut auf der Gewebeoberfläche gleitend auszubilden, also z.B. kufenförmig, wie in den Fig. 1 bis 3 sowie 7 bis 9 dargestellt, oder mit einer Rolle, wie in den Fig. 4 bis 7 dargestellt.

Abweichend von der in den Fig. 1 bis 3 dargestellten Ausführungsform kann der Drahtbügel 9 beispielsweise auch in der Mitte geteilt aus zwei jeweils eine Kufe ausbildenden Teilen bestehen.

In den beschriebenen Ausführungsformen des Distanzhalters ist dieser vorgesehen, um der Schneidschlinge Distanz nach vorn sowie zur Begrenzung der Schneidtiefe nach unten zu geben. Der Distanzhalter kann die Schneidschlinge aber auch seitlich überragen und somit verhindern, daß die Schneidschlinge seitlich benachbart liegende Körperteile berührt.

Durch die Begrenzung der Schneidtiefe verringert sich bei den dargestellten Schneidelektroden die Resektionsleistung. Es kann daher bei Beginn der Operation, wenn größere Volumina zu entfernen sind, mit einer normalen Schneidelektrode ohne Distanzhalter gearbeitet werden. Diese kann dann gegen eine erfindungsgemäße Schneidelektrode mit Distanzhalter ausgewechselt werden, um die letzten Feinarbeiten in der Nähe zu schonender Organe durchzuführen, beispielsweise um im Falle der Prostataresektion die letzten Reste des Prostatagewebes 6 von der Oberfläche der Kapsel 7 zu entfernen, ohne in diese einzuschneiden. Erfindungsgemäße Schneidelektroden mit Distanzhalter können dem Operateur als Sortiment mit unterschiedlicher Schneidtiefe bereitgestellt werden.

Die Erfindung kann auch bei um die Schaftachse rotierend angetriebenen Schneidelektroden eingesetzt werden, bei denen die Schneidschlinge im wesentlichen in einer durch die Schaftachse verlaufenden Ebene liegt. Auch hier wäre ein Distanzhalter vorzusehen, der bis vor die Schneidschlinge ragt und so weit nach unten bzw. ring- oder scheibenförmig nach allen Seiten von der Drehachse ausgehend, daß die Schneidtiefe der rotierenden Schlinge begrenzt wird.

## Patentansprüche

1. Schneidelektrode für medizinisches Resektoskope, mit einem in Achsrichtung des Resektoskopschaftes bewegbaren isolierten Schlingenträger, dessen distales Ende eine nichtisolierte Schneidschlinge trägt, die sich von, Ende des Schlingenträgers quer zur Achsrichtung bis zu einer die maximale Schnittiefe bestimmenden Tiefe erstreckt, dadurch gekennzeichnet, daß am Schlingenträger (4, 4') ein Distanzhalter (9, 9', 9'') vorgesehen ist, der sich distal vor die Schneidschlinge (5, 5') sowie nach unten bis zu einer geringeren Tiefe als die der Schneidschlinge erstreckt.

2. Schneidelektrode nach Anspruch 1, dadurch gekennzeichnet, daß der Distanzhalter (9, 9', 9'') als isolierter Drahtbügel ausgebildet ist.

3. Schneidelektrode nach einem der vorhergehenden Ansprüche, dadurch gekennzeichnet, daß die Gebiete (10) größter Tiefe des Distanzhalters (9) in Achsrichtung gleitbar kufenartig nach oben gekrümmt ausgebildet sind.

4. Schneidelektrode nach einem der Ansprüche 1 oder 2, dadurch gekennzeichnet, daß an der Stelle größter Tiefe des Distanzhalters (9') eine quer zur Achsrichtung gelagerte Rolle (11) vorgesehen ist.

5. Schneidelektrode nach einem der Ansprüche 2 oder 3, dadurch gekennzeichnet, daß der Drahtbügel (9) zwei tiefste Punkte (10) aufweist, die in einem seitlichen Abstand angeordnet sind, der im wesentlichen der Breite der Schneidschlinge (5) entspricht.

6. Schneidelektrode nach Anspruch 5, dadurch gekennzeichnet, daß der Drahtbügel (9) zwischen den tiefsten Punkten hochgewölbt ist.

## Claims

1. Cutting electrode for a medical resectoscope with an insulated loop carrier which is movable in the axial direction of the resectoscope shaft and whose distal end carries a non-insulated cutting loop which extends from the end of the loop carrier transverse to the axial direction to a depth which determines the maximum cutting depth, characterised in that provided on the loop carrier (4, 4') there is a spacer (9, 9', 9'') which extends distally in front of the cutting loop (5, 5') and downwardly to a lesser depth than that of the cutting loop.

2. Cutting electrode as claimed in Claim 1, characterised in that the spacer (9, 9', 9'') is constructed as an insulated wire strap.

3. Cutting electrode as claimed in one of the preceding claims, characterised in that the regions (10) of greatest depth of the spacer (9) are curved upwardly in the manner of runners to be slidable in the axial direction.

4. Cutting electrode as claimed in one of Claims 1 or 2, characterised in that provided at the point of greatest depth of the spacer (9') there is a roller (11) mounted transverse to the axial direction.

5. Cutting electrode as claimed in one of Claims 2 or 3, characterised in that the wire strap (9) has two deepest points (10) which are disposed at a lateral spacing which substantially corresponds to the breadth of the cutting loop (5).

6. Cutting electrode as claimed in Claim 5, characterised in that the wire strap (9) is curved upwardly between the deepest points.

## Revendications

1. Electrode coupante pour résecteurs, comprenant un support d'anse isolé, mobile dans la direction de l'axe du fût du résecteur, support dont l'extrémité distale porte une anse coupante non isolée qui s'étend depuis l'extrémité du support, transversalement à la direction axiale, jusqu'à une profondeur déterminant la profondeur de coupe maximale, électrode caractérisée en ce qu'un élément d'écartement (9, 9', 9'') est prévu sur le support d'anse (4, 4'), élément d'écartement qui s'étend, en direction distale, jusque devant l'anse coupante (5, 5'), ainsi que, vers le bas, jusqu'à une profondeur qui est inférieure à celle de l'anse coupante.

2. Electrode coupante selon la revendication 1, caractérisée en ce que l'élément d'écartement (9, 9', 9'') est réalisé comme un étrier en fil isolé.

3. Electrode coupante selon une des revendications précédentes, caractérisée en ce que les zones (10) de profondeur maximale de l'élément d'écartement (9) sont recourbées vers le haut, à la façon de patins, pour qu'elles puissent glisser en direction axiale.

4. Electrode coupante selon la revendication 1 ou 2, caractérisée en ce qu'un galet (11), monté rotatif autour d'un axe transversal à la direction axiale, est prévu à l'endroit de profondeur maximale de l'élément d'écartement (9').

5. Electrode coupante selon la revendication 2 ou 3, caractérisée en ce que l'étrier en fil (9) présente deux points (10) situés le plus bas et mutuellement espacés latéralement d'une distance correspondant essentiellement à la largeur de l'anse coupante (5).

6. Electrode coupante selon la revendication 5, caractérisé en ce que l'étrier en fil (9) est bombé vers le haut entre les points les plus bas.
